# EUROPEAN PATENT SPECIFICATION

(11) **EP 1 893 740 B1**
(45) Date of publication and mention of the grant of the patent: **25.03.2009**
(21) Application number: 06743397.9
(22) Date of filing: 02.05.2006
(51) Int. Cl.: C12M 1/30

(54) **DEVICE FOR THE WITHDRAWAL, COLLECTION AND TRANSPORT OF BIOLOGICAL SPECIMENS**
VORRICHTUNG ZUR ENTNAHME, ZUM SAMMELN UND ZUM TRANSPORT BIOLOGISCHER PROBEN
DISPOSITIF SERVANT A PRELEVER, RECUEILLIR ET TRANSPORTER DES SPECIMENS BIOLOGIQUES

(30) Priority: 08.06.2005 IT MI20051057
(43) Date of publication of application: 05.03.2008
(73) Proprietor: Copan Innovation Limited, Limerick (IE)
(72) Inventor: TRIVA, Daniele, I-25073 Bovezzo (IT)
(74) Representative: Appoloni, Romano
(86) International application number: PCT/EP2006/061975
(87) International publication number: WO 2006/131424

(56) References cited:
- EP-A- 0 366 826
- EP-A- 0 643 131
- WO-A-20/04086979
- DE-A1- 10 205 709
- FR-A- 2 540 513
- GB-A- 2 358 061
- US-A- 5 163 441
- PATENT ABSTRACTS OF JAPAN vol. 2000, no. 16, 8 May 2001 (2001-05-08) & JP 2001 000170 A (KIKKOMAN CORP), 9 January 2001 (2001-01-09)

## Description

### Field of the invention

The present invention relates to a device for the withdrawal, collection and transport of biological specimens.

### Prior art

In the clinical and diagnostic analysis field, swabs of organic maternal for the withdrawal of biological specimens are known, consisting essentially of a cylindrical rod whose tip is provided with a means for absorbing the specimen to be analysed. If the specimen is to be sent for testing by culturing the microorganisms collected by withdrawal, the swab bearing the specimen is immersed in a sterile test tube.containing culture medium immediately following withdrawal, for appropriate preservation during transport thereof to the analytical laboratory and while awaiting the analysis itself. The test tube is re-sealable hermetically with a cap to guarantee the sterility of the contents. Swabs of this type are for example described in EP0643131 or in W02004/086979 by the same Applicant.

Generally, in this type of device, the specimen is -subject to contamination risks both during the withdrawal step and the step of opening the test tube for implementing the analysis and, notwithstanding the amount of care used, it is not improbable that there is inadvertently a contact between the swab and, for example, the non-sterile environment or the hands of the nurse who carries out the withdrawal or of the operator who undertakes the analysis.

In EP0366826 for example a device is described in which the rod of the swab is breakable such that, after withdrawal for example from the oral cavity, the rod is broken so that the swab can be inserted into the test tube which is then resealed with a cap equipped with a means to grasp the end of the broken rod. Therefore when carrying out the analysis the test tube has merely to be opened, knowing that the cap carries with it the specimen with no possibility of contact between this latter and the operator, who has only to handle the cap without risk of touching the swab.

FR 2540513 describes a medical type swab container for aseptic storage and transportation of a sample of micro-organism, in particular bacteria for laboratory analysis.

The swab is fixed in the inner face of a plastic stopper (a) sealed in the open end of a sterile, plastic tube with a clambing ring (b). After sampling the swab can be pushed further into the tube so that it ruptures a plastic membrane (diaphgram) and breaks into a chamber containing a conservation medioum for micro-organism.

### Summary of the invention

The present invention proposes a completely different solution to the problem of possible contamination of the specimen withdrawn by a swab of the aforedescribed type.

To this end, the present invention proposes a device for the withdrawal, collection and transport of biological specimens characterised by comprising a test tube equipped with a cap for sealed closure comprising a membrane constructed of a soft material impermeable to liquids and pierceable by a suitable specimen withdrawal means.

The invention relates to the device in its entirety that comprise it, i.e. cap and test tube and possibly a swab for the withdrawal of said specimen.

With the aim of better understanding the characteristics and advantages of the invention, a non-limiting embodiment is described hereinafter, with reference to the figures of the accompanying drawings.

### Brief description of the drawings

Figure 1 shows a longitudinal cross-section of the device of the invention.
Figure 2 shows a longitudinal cross-section of a detail of the device of the invention, i.e. said cap, in a variation of the invention.

### Detailed description of the invention

With reference to said figures, a device 10 for the withdrawal, collection and transport of biological specimens comprises a test tube 11 equipped upperly with a cap 12 and containing a culture medium 21, for example in liquid form.

The device comprises a swab 13 consisting of a rod 15, possibly breakable, terminating with a tip 16 bearing a suitable absorbent means for the withdrawal of the specimen to be analysed.

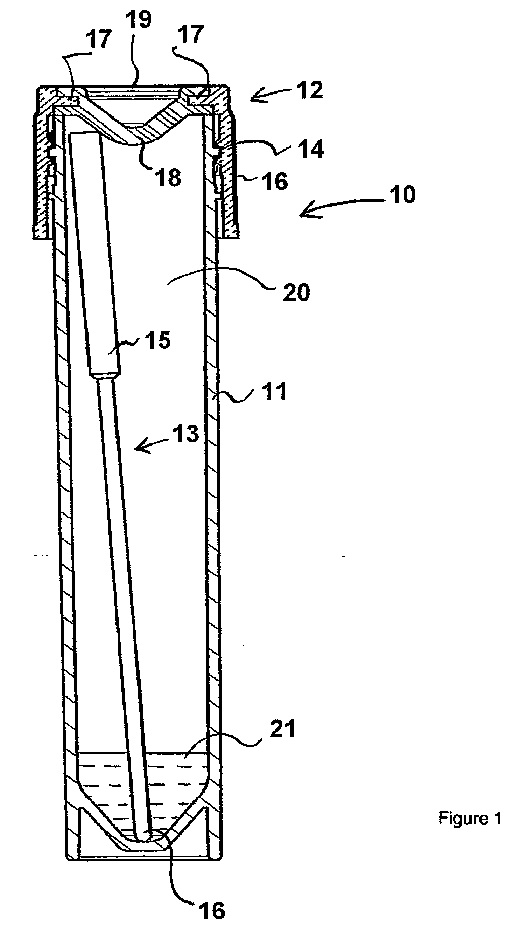

In the example shown in fig. 1, said cap 12 consists of an annular sleeve 16 screwable onto the upper end of the test tube by the effect of a thread 14. In its upper part, which remains unengaged when the test tube is in its closed position after screwing on, said sleeve is internally shaped with a continuous or discontinuous circular projection 17 suitable for sealedly coupling to the edge of a liquid-impermeable pierceable discoid membrane 18 of soft material, for example of silicone rubber, so that the coupled sleeve 16 and membrane 18 constitute a means of sealed closure of the test tube 11 when the cap is screwed on.

Said membrane 18 preferably has a concave shape, as can be deduced from the substantially U or V shaped section shown in the figures.

It is upperly protected by a film 19 of a plastic material or another suitable material, such as aluminium, applied to the upper edge of the sleeve 16, which acts as a guarantee seal for the cap.

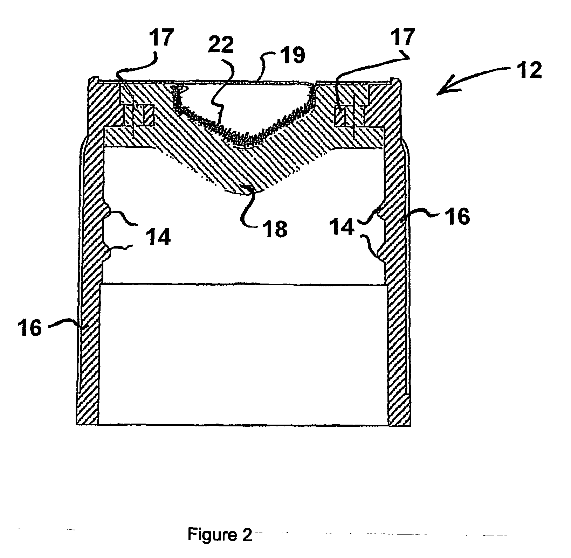

In a variant of the invention shown particularly in fig. 2, the liquid-impermeable pierceable membrane 18 of soft material, for example of silicone rubber, is coated upperly with a layer 22 of a liquid absorbent material, for example deposited by flocking.

The operation of the device shown in the example is the following: after withdrawing the specimen from the patient by means of the swab 13, the test tube 11 is opened by unscrewing the cap 12 and the swab 13 is inserted so that the tip 16 bearing the withdrawn specimen is immersed in the culture medium 21. If the swab is sufficiently long to require it, the breakable rod 15 is firstly broken so that the test tube containing the swab can be easily resealed by re-screwing the cap 12.

Due to the concave shape of the membrane 18, the rod of the swab falls obliquely into the test tube and remains substantially in this position as shown in fig. 1, thus freeing within the test tube the space 20 which develops about the longitudinal axis thereof.

In the arrangement of figure 1, the device 10 can be preserved and transported for analysis. For implementing this latter, the operator can insert a micropipette by pressing its tip against the centre of the membrane 18 until it yields to the pressure and is pierced. The pipette can then penetrate into the test tube 11 along the space 20 until it reaches the culture medium 21 containing the specimen to be analysed. The pipette, having withdrawn the desired volume of culture medium with specimen, is then removed from the test tube to carry out the analysis, without there having occurred any contamination since only the pipette has come into contact with the specimen.

In the variant of the invention in fig. 2, said layer of absorbent material 22 also allows any liquid spillages to be absorbed, even in the form of droplets, that may occur on removing the micropipette from the test tube due to friction on passing through the perforation opening in membrane 18. This fully achieves the advantage of avoiding uncontrolled spillage of the specimen and consequent contamination.

As can be understood from the preceding description, the present invention enables the initially stated object to be effectively attained, independently of the particular embodiment.

Numerous structural modifications of the aforedescribed example can be made to the invention, for example relating to the material and shape of the test tube and cap, and their constituent parts. In addition the dimensions of the rod of the swab and the relative positioning within the test tube can be different from those shown in the illustrative drawing, provided that the interior space of the test tube which develops about its longitudinal axis is left free, a space in which the pipette must be able to operate for withdrawing the specimen to be analysed. The culture medium could be in a form other than liquid, for example a low viscosity gel able to be still withdrawn with a pipette or by another suitable means.

## Claims

1. A device (10) for the withdrawal, collection and transport of biological specimens, **characterised by** comprising a test tube (11) equipped with a cap (12) comprising a membrane (18) constructed of a soft material impermeable to liquids and pierceable by a suitable specimen withdrawal means.

2. A device as claimed in claim 1 **characterised in that** said cap (12) consists of an annular sleeve (16) which **in that** upper part not engaged by the test tube when in the closed position is provided with means (17) suitable for sealedly coupling to said membrane (18).

3. A device as claimed in claim 2 **characterised in that** said means (17) consists of a continuous or discontinuous circular projection suitable for sealedly coupling to the edge of a discoid membrane (18).

4. A device as claimed in claim 2 **characterised in that** said annular sleeve (16) is screwable onto the upper end of said test tube by the effect of a thread (14).

5. A device as claimed in claim 1 **characterised in that** said membrane (18) has a concave shape.

6. A device as claimed in claim 1 **characterised in that** said membrane (18) is of silicone rubber.

7. A device as claimed in claim 1 **characterised in that** said membrane (18) is coated upperly with a layer (22) of a liquid absorbent material.

8. A device as claimed in claim 7 **characterised in that** said membrane (18) is coated upperly with a layer (22) of a liquid absorbent material deposited by flocking.

9. A device as claimed in claim 1 **characterised in that** said membrane is surmounted by a protective film (19) applied to the upper edge of the cap (12).

10. A device as claimed in claim 1 **characterised by** comprising a test tube (11) equipped with a cap (12) and a swab (13) for specimen withdrawal from a patient.

## Patentansprüche

1. Vorrichtung (10) für die Entnahme, das Sammeln und den Transport biologischer Proben, **dadurch gekennzeichnet, dass** sie ein Test-Rohr (11) umfasst, das mit einer Kappe (12) ausgestattet ist, die eine Membran (18) umfasst, die aus einem weichen Material aufgebaut ist, das undurchlässig für Flüssigkeiten ist und durch eine geeignete Proben-Entnahme-Einrichtung durchstechbar ist.

2. Vorrichtung nach Anspruch 1, **dadurch gekennzeichnet, dass** die Kappe (12) aus einer ringförmigen Hülse (16) besteht, die in dem oberen Teil, der nicht mit dem Test-Rohr in Eingriff ist, wenn sie in der geschlossenen Position ist, mit einer Einrichtung (17) versehen ist, die für ein dichtschließendes Koppeln mit der Membran (18) geeignet ist.

3. Vorrichtung nach Anspruch 2, **dadurch gekennzeichnet, dass** die Einrichtung (17) aus einem kontinuierlichen oder diskontinuierlichen kreisförmigen Vorsprung besteht, der zum dichtschließenden Koppeln mit dem Rand einer scheibenförmigen Membran (18) geeignet ist.

4. Vorrichtung nach Anspruch 2, **dadurch gekennzeichnet, dass** die ringförmige Hülse (16) auf das obere Ende des Test-Rohrs durch die Wirkung eines Gewindes (14) schraubbar ist.

5. Vorrichtung nach Anspruch 1, **dadurch gekennzeichnet, dass** die Membran (18) eine konkave Form hat.

6. Vorrichtung nach Anspruch 1, **dadurch gekennzeichnet, dass** die Membran (18) aus Silicon-Kautschuk besteht.

7. Vorrichtung nach Anspruch 1, **dadurch gekennzeichnet, dass** die Membran (18) auf der Oberseite mit einer Schicht (22) aus einem flüssigen absorbierenden Material beschichtet ist.

8. Vorrichtung nach Anspruch 7, **dadurch gekennzeichnet, dass** die Membran (18) auf der Oberseite mit einer Schicht (22) aus einem flüssigen absorbierenden Material beschichtet ist, das durch Flocken abgeschieden wurde.

9. Vorrichtung nach Anspruch 1, **dadurch gekennzeichnet, dass** die Membran überzogen wird durch einen Schutzfilm (19), der auf den oberen Rand der Kappe (12) aufgebracht wurde.

10. Vorrichtung nach Anspruch 1, **dadurch gekennzeichnet, dass** sie ein Test-Rohr (11) umfasst, das mit einer Kappe (12) und einem Tupfer (13) für ein Abnehmen einer Probe von einem Patienten ausgerüstet ist.

## Revendications

1. Dispositif (10) pour le prélèvement, la collecte et le transport d'échantillons biologiques, **caractérisé en ce qu'**il comprend un tube à essai (11) muni d'un capuchon (12) comprenant une membrane (18) réalisée en un matériau souple imperméable aux liquides et transperçable par un moyen de prélèvement d'échantillon approprié.

2. Dispositif selon la revendication 1, **caractérisé en ce que** ledit capuchon (12) est constitué par un manchon annulaire (16) qui, dans la partie supérieure ne s'engageant pas sur le tube à essai, en position d'obturation, est muni d'un moyen (17) approprié pour le raccorder de manière étanche à ladite membrane (18).

3. Dispositif selon la revendication 2, **caractérisé en ce que** ledit moyen (17) est constitué par une lèvre circulaire continue ou discontinue appropriée pour la raccorder de manière étanche au bord d'une membrane discoïde (18).

4. Dispositif selon la revendication 2, **caractérisé en ce que** ledit manchon annulaire (16) peut se visser sur l'extrémité supérieure dudit tube à essai au moyen d'un filetage (14).

5. Dispositif selon la revendication 1, **caractérisé en ce que** ladite membrane (18) a une forme concave.

6. Dispositif selon la revendication 1, **caractérisé en ce que** ladite membrane (18) est en caoutchouc silicone.

7. Dispositif selon la revendication 1, **caractérisé en ce que** ladite membrane (18) est enduite sur son dessus d'une couche (22) d'un matériau liquide absorbant.

8. Dispositif selon la revendication 1, **caractérisé en ce que** ladite membrane (18) est enduite sur son dessus d'une couche (22) d'un matériau liquide absorbant déposé par flocage.

9. Dispositif selon la revendication 1, **caractérisé en ce que** ladite membrane est surmontée d'un film protecteur (19) appliqué sur le bord supérieur du capuchon (12).

10. Dispositif selon la revendication 1, **caractérisé en ce qu'**il comprend un tube à essai (11) muni d'un capuchon (12) et d'un tampon (13) pour prélever un échantillon chez un patient.
